# EUROPEAN PATENT SPECIFICATION

(11) **EP 1 083 232 B1**
(45) Date of publication and mention of the grant of the patent: **23.02.2005**
(21) Application number: 99117771.8
(22) Date of filing: 09.09.1999
(51) Int. Cl.: C12N 15/87, A61K 48/00, A61K 38/00, A61P 31/12

(54) **Transfer of mRNA using polycationic compounds**
Transfer von mRNAs unter Verwendung von polykationischen Verbindungen
Transfert de mARN á l'aide de composés polycationiques

(43) Date of publication of application: 14.03.2001
(73) Proprietor: CureVac GmbH, 72076 Tübingen (DE)
(72) Inventor: Jung, Günther, Prof.Dr., 72076 Tübingen (DE); Hoerr, Ingmar, 72070 Tübingen (DE); Rammensee, Hans-Georg, Prof.Dr., 72070 Tübingen (DE); Obst, Reinhard, Dr., Boston, MA 02215 (DE)
(74) Representative: Graf von Stosch, Andreas, Dr. rer. nat.

(56) References cited:
- WO-A-93/04701
- FR-A- 2 676 072
- US-A- 5 945 400
- FISHER K J ET AL: "The transmembrane domain of diphtheria toxin improves molecular conjugate gene transfer." BIOCHEMICAL JOURNAL, vol. 321, no. 1, 1 January 1997 (1997-01-01), pages 49-58, XP000867796
- DUBES G R ET AL: "RAPID EPHEMERAL CELL SENSITIZATION AS THE MECHANISM OF HISTONE- INDUCED AND PROTAMINE-INDUCED ENHANCEMENT OF TRASNFECTION BY POLIOVIRUS RNA" PROTOPLASMA,AT,SPRINGER VERLAG, VIENNA, vol. 96, 1 January 1978 (1978-01-01), pages 209-223, XP000575919 ISSN: 0033-183X
- FORG P ET AL: "Superiority of the ear pinna over muscle tissue as site for DNA vaccination." GENE THERAPY, vol. 5, no. 6, June 1998 (1998-06), pages 789-797, XP000867787
- MURAKAMI M ET AL: "HIGH-LEVEL EXPRESSION OF EXOGENOUS GENES BY REPLICATION-COMPETENT RETROVIRUS VECTORS WITH AN INTERNAL RIBOSOMAL ENTRY SITE" GENE: AN INTERNATIONAL JOURNAL ON GENES AND GENOMES,GB,ELSEVIER SCIENCE PUBLISHERS, BARKING, vol. 202, 1 January 1997 (1997-01-01), pages 23-29, XP000749204 ISSN: 0378-1119
- BLACKBURN P ET AL: "Ribonuclease inhibitor from human placenta. Purification and properties." JOURNAL OF BIOLOGICAL CHEMISTRY, vol. 252, no. 16, August 1977 (1977-08), pages 5904-5910, XP000867786
- KILPATRICK M W ET AL: "Delivery of a hammerhead ribozyme specifically down-regulates the production of fibrillin-1 by cultured dermal fibroblasts." HUMAN MOLECULAR GENETICS, vol. 5, no. 12, December 1996 (1996-12), pages 1939-1944, XP002129886

## Description

The invention relates to the use of in RNA for the preparation of a vaccine for the tranfer of mRNA particularly into cells.

A lot of methods have been developed in the past decades in order to transfer nucleic acids into cells and especially into organisms. In particular, transfer methods for eukaryotic cells were taken into account in the last years. In this connection methods which are suitable in the context of gene therapy are of particular interest.

Gene therapy is a medical procedure that treats a disorder by replacing or counteracting a faulty gene. Such a therapy demands a high measure of safety precautions to minimize risks and dangers for the treated person.

The common approach for the research in the field of gene therapy is the use of DNA to introduce the needed genetic information into the cell. There are several methods known which are useful and comparatively effective in delivering DNA into the cell, e.g. calcium phosphate transfection, Polybren transfection, protoplast fusion, electroporation, , microinjection or lipofection. Especially lipofection has been proved to be a useful tool.

One method especially applied in the field of gene therapy is the use of DNA-viruses as vehicles for the DNA. Viruses have the important advantage that they are infective by themselves. Therefore, they have no problems in entering the cell. On the other hand, by working with viruses a certain risk always remains. It is not possible to exclude that the virus spreads uncontrollably in the organism, in spite of the fact that obviously the used viruses have been genetically engineered to exclude this risk as far as possible.

Usually, the DNA delivered into the cell integrates to a certain extent into the genome of the transfected cell. This could be a desired effect because this could possibly enable a long-persisting effect of the introduced DNA. On the other side, integration into the genome involves one main risk of gene therapy. By integration the introduced DNA could cause an insertion in an intact gene of the transfected cell, i.e. a mutation which disturbes the function of that particular gene. Hereby, an important enzyme, for example, could be knocked out, and the cell possibly looses its viability. Since the integration into the genome is a statistic event only a portion of the cells will die due to the integration of the delivered DNA causing lethal insertion mutations. Therefore, lethal insertion mutations will not lead to extreme drastic after-effects for the organism, in general. It is more fatal that due to the mutation caused by the insertion of the DNA the starting point for the development of a cancer could be given. For example, by destroying the gene encoding a regulatory factor for proliferation the cell can show unlimited division. Such an abnormal proliferative cell could entail a cancerous disease with all known bad consequences for the concerned patient. For this it could be sufficient that only one single cell is converted into a cancerous cell which is the starting point for the cancer development.

When working with DNA, it is mandatory to connect the gene to be introduced with a strong promoter, e.g. the viral CMV-Promotor, in order to achieve a sufficient translation rate. The potential risks of such promoters are not exactly known , but it is not possible to exclude the insertion of said promoters in the genome of the treated cell with fatal consequences for the regulation of gene expression.

Another dangerous aspect of using DNA in gene therapy is the induction of pathogenic anti-DNA antibodies within the treated organism causing a fatal immune defence.

Due to these reasons in the past several considerations and attempts were made in order to circumvent the risks of DNA, especially of uncontrollable insertion of DNA in the field of gene therapy. A solution of this problem would be the use of RNA instead of DNA as a carrier of genetic information

Fisher et al. (Biochemical Journal, vol. 321, No. 1, 1997, pp. 49 - 58) describe the transfer of complexes consisting of luciferase mRNA, ASOR-polylysine and a conjugate consisting of maltose-binding protein, the diphtheria toxin transmembrane domain and a polylysine into cells.

Dubes et al. (Protoplasma 96, 1978, pp. 209 - 223) disclose the transfection and enhancement by protamine and histone using poliovirus RNA and sheet cultures of the CLI line of chimpanzee liver cells.

Murakami et al. (Gene 202 (1997) 23 - 29) report the construction of two types of Rous sarcoma virus (RSV)-based replication-competent avian retrovirus vectors. It is shown that exogenous genes can be high level expressed by replication-competent retrovirus vectors with an internal ribosome entry side.

WO 96/25508 (Rhone-Poulenc Rorer) discloses pharmaceutical compositions useful for transfecting an nucleic acid and characterized in that it contains in addition to any nucleic acid at least one transfection agent and a compound causing the condensation of said nuleic acid. This compound is totally or partly derived from a histone, a nucleoline, a protamine, and/or a derivate thereof. Furthermore, the use of such compositions for transferring nucleic acid in vivo and in vitro is shown.

RNA by itself is not able to integrate into the genome. Therefore, by the use of RNA mutations caused by insertion will not take place. One further advantage of the use of RNA is that RNA could be directly translated by the translation machinery of the cell. The step of transcription of DNA into mRNA as it is needed when using DNA for transfection is not necessary. Therefore, the genetic information is directly translated into the desired gene product, e.g. an enzyme, without delay. An appearance of anti-RNA antibodies, which could cause problems in respect to clinical applications, were not observed until now.

In order to ensure that the RNA is identified and correctly translated within the transfected cell it is necessary to work with RNA compatible with the eukaryotic translation system. By transfecting messenger RNA (mRNA) which contains a 5'-cap and a polyA-tail and possibly further appropriate translation signals like e.g. a ribosomal binding site it is secured, that the genetic information is correctly used by the cell.

An important applicability of RNA is the antisense approach. Here a RNA complementary to the target mRNA is transferred into the cell. Due to complemetarity both RNAs interact with each other and thereby the translation of the target mRNA is inhibited. By this technology the expression of a special undesired protein can be suppressed.

Another important field of application of transfer of RNA are the so-called ribozymes. Ribozymes are RNA molecules with enzymatic activity which attack desired targets in the cell very specifically.

In summary, RNA would be a very efficient tool in the field of gene therapy and in the field of molecular research on the whole.

So far RNA was considered to be very problematical in laboratory and clinical handling. Since RNA is very prone to hydrolyses by ubiquitous ribonucleases it is not stable in solution. Even smallest contaminations of ribonucleases are sufficient to degrade RNA completely. Therefore, gene therapy approaches involving nucleic acids have until now focused on DNA which is much easier in handling (Ref. 1).

Nevertheless, it has been shown that naked RNA injected into mouse skeletal muscle does lead to gene expression in vivo (Ref. 2) and that cytotoxic T cells can be primed in vivo with liposome encapsulated mRNA which so far could be prepared by an extremly complicated procedure (Ref. 3).

Recently, it has been shown by Ying et al. that a self-replicating RNA vaccine is a potentially useful candidate for the treatment of patients with cancer (Ref. 23). Vaccination is based on introducing an antigen, in this case the genetic information (DNA or RNA) for an antigen, into the organism, especially into the cell. The genetic information is translated into the antigen, i.e. into a certain peptide or protein and thereby an immune response directed against this peptide or protein is stimulated. In respect to the treatment of cancer this can be achieved by introducing the genetic information of cancer antigens, e.g. proteins solely expressed by cancerous cells. Then, these cancer antigens are expressed in the organism and an immune response is elicited which is efficiently directed against the cancerous cells eliminating them. Due to the extreme sensitivity and the very low stability of RNA the immunogenicity of this nucleic acid is normally very low. The authors of the above-mentioned article enhanced the immunogenicity of the nucleic acid by making it "self-replicating". This was accomplished by using a gene encoding RNA replicase polyprotein derived from the Semliki forest virus in combination with a RNA encoding for a model antigen.

The main disadvantage of the approach of Ying et al. could be that the viral enzyme RNA replicase is necessary to enhance the efficacy of the RNA introduced. The effects of this foreign enzyme in the organism treated are not known. This enzyme is able to replicate RNA unspecifically, i.e. it replicates RNA from viruses which could enter the organism by normal infections at random, thereby increasing the risk of dangerous infections drastically. This makes the approach of Ying et al. risky for clinical applications.

Another disadvantage when using replicase is that cells transfected with this enzyme show apoptosis, i.e. cell death, within about 24 h. These short-living cells are not able to produce the desired gene product in sufficient amounts. That is another reason why this approach is not suited for applications in the field of gene therapy where relatively long-persisting expression of the target gene has to be achieved.

Therefore, the invention has the object to provide an efficient way for introducing mRNA into cells, especially into (living) organisms which circumvents the above-mentioned problems and which allows its use for the preparation of a vaccine. This object is solved by claim 1. Special embodiments of the inventive subject-matter are depicted in the dependent claims 2 to 13. The wording of all claims is hereby made to the content of the specification by reference.

The invention is based on the surprising results of the inventors that mRNA could be efficiently transferred into cells evoking an immune response if the mRNA is associated or boundto a cationic peptide or protein and, thereby, forms a complex. The use of protamine as a polycationic nucleic acid binding protein is especially effective. Use of other cationic peptides or proteins like poly-L-lysine or histone is also possible.

Protamines are basic (cationic) small proteins of molecular weights of about 4,200 Da. They are found tightly associated with DNAs in fish spermatozoa. Nearly two-thirds of the amino acid residues in protamines are basic, and these basic residues are usually found clustered, four or five in a sequence.

In the Sixties and Seventies the effect of basic proteins like protamine on the infectivity of viruses was investigated. It was shown that basic proteins provoked a sensitization of cells in respect to infectivity of viral RNA (Refs 21 and 22). In 1978 it was shown that protamine forms a complex with double stranded tRNA (Ref. 9). These research results were not considered to be important for the field of gene therapy.

The inventors now clearly showed the protective effect of basic proteins like protamine on the critical stability of single stranded RNA, especially mRNA. Based on these results a composition used a method for the transfer of mRNA into cells was developed, wherein the mRNA is transferred in the form of a complex or a comparable associated structure between at least one mRNA and at least one cationic, especially polycationic peptide or protein. Complex formation by essentially ionic interactions has the decisive effect of stabilizing the mRNA and preventing it from degradation. Thus, the genetic information is delivered into the cell without the drastic loss of efficacy which normally makes the transfer of functional RNA into cells impossible.

In general, all cationic, especially polyicationic, peptides or proteins should be suited for the use for the preparation as a vaccine in a complex with mRNA. In particular, nucleic acid binding peptides or proteins show good results. In a preferred embodiment the cationic/basic peptides are short and possess a length of up to 100 amino acids. Nevertheless, the invention comprises use of peptides or proteins which are larger. Usable peptides or proteins are preferably provided by purification of naturally occuring or recombinantly expressed peptides or proteins, since such substances are commonly available. Another possibility is to produce the substances by chemical synthesis. Especially suited are protamine, poly-L-lysine and histone.

Side-effects of these proteins or peptides, especially regarding gene therapy, are negligible, since the above mentioned peptides or proteins are not immunogenic. Therefore, the treatment of a patient with said peptides or proteins will not evoke an immune response against the administered composition comprising mRNA and the cationic peptide or protein.

The complex comprising mRNA and the cationic peptide or protein is preferably administered into subarachnoid spaces, peripheral lymphatic nodes, tumour tissues and/or cartilaginous tissues. Especially promising results were obtained by administration into cartilaginous tissues. In one embodiment of the invention the complex is administered into aural tissues, especially into the ear pinna. The applicability in aural tissues is of particular importance since conventional methods, like liposome-mediated nucleic acid transfer into aural tissues is not possible.

For delivering the complexed mRNA into the cell, especially into the organism, e.g. animals or humans, there are several possibilities. In preferred embodiments of the complex is delivered by injection, particularly by intradermal, intramuscular, intravenous, subcutaneous and/or intranasal injection.

The mRNA to be transferred encodes at least one antigen. Subsequent to transfer of the nucleic acid the encoded antigen is translated by the translation machinery of the transfected cell. This antigen evokes an immune response within the organism. This response is usable to treat a certain disease, for example to treat tumours or cancer. A specific protein or peptide of the target cells to be eliminated is chosen as antigen. Another possibility is to deliver the genetic information for more than one antigen into the cell. Preferably, collections of mRNAs are transferred in order to achieve a strong immune response. Clearly, the invention comprises the transfer of genetic information encoding e.g. enzymes or other peptides or proteins or collections thereof. Furthermore, the invention comprises the delivery of antisense RNA or collections thereof into the cell.

In a preferred embodiment of the invention the collection of mRNAs is achieved by preparing a RNA library derived from e.g. tumour tissue. The collection of mRNAs could be built up by fractionated or unfractionated RNA libraries.

In an especially preferred embodiment of the invention the aim of introducing mRNA encoding antigens into the cell is to stimulate cytotoxic T cells for example. This is achieved by the presentation of antigenic peptides in connection with MHC class I complex on the surface of antigen presenting cells, which have been transfected by mRNA. This way of evoking an immune response is similar to the stimulation by viral pathogens. Simultaneously, a humoral immune response is achieved by stimulation of T helper cells due to the immunization with mRNA. Thereby, the immunization with mRNA leads to the possibility of treatment of viral infections up to the treatment of cancer. Especially the treatment of cancer is performable by immunization with an unfractionated and/or fractionated RNA library produced from tumour tissue. This is possible even in the case of very small sample amounts of tumour tissue, since it is possible to generate a cDNA library which can be amplificated and stored, if needed so. From this cDNA library RNA can be transcribed in vitro in high amounts which can be used for vaccination in the inventive manner

In an especially preferred embodiment of the invention the mRMA used as part of a composition for the preparation of a vaccine comprises sequences, especially at the 5'- and/or 3'-terminal end, which are not translated within the cell. These additional sequences are especially preferred in order to achieve a further stabilization of the mRNA. In clinical applications such a further stabilization of RNA could be desirable.

For example in order to increase the translational efficacy of the transferred mRNA it could be advantageous to use a mRNA which comprises at least one internal ribosomal entry site (IRES). Such a sequence promotes the entry and binding of the mRNA into the ribosome whereby the translation rate is increased.

In a preferred embodiment of the invention the transferred RNA comprises two or more genes, e.g. a gene encoding for an antigen and a gene encoding for a factor, e.g. a cytokine, which stimulates the immune response or costimulating surface receptors. With respect to such multifunctional RNAs IRES could be used as well-suited components when constructing the corresponding vectors.

In comparison with the transfection using DNA it was shown by the inventors that the transfer of RNA using the inventive method is much more effective. About 1 µg RNA per 30 g living weight was shown to be effective in evoking a specific cytotoxic T cell response in mouse whereas 50 µg DNA per 30 g living weight were necessary to evoke the same effect. Thus, the inventive method has the considerable advantage that much less genetic material is needed to obtain the desired effect thereby reducing the costs.

Therefore, the invention comprises a complex which is formed by at least one cationic peptide or protein and at least one mRNA which is used for the preparation of a vaccine. Regarding the features of this complex reference is made to the above description.

At least one inventive complex as described above is part of a composition used for the preparation of a vaccine. Further ingredients of the composition are preferably buffer solutions and/or further components which promote the stability of the mRNA. Preferably the composition comprises at least one RNase inhibitor. RNase inhibitors are commercially available and are widely used in laboratory practice. One example of a suitable RNase inhibitor is the RNase inhibitor of human placenta. It is also possible to stabilize the RNA by other treatments, e.g. special salt conditions, as they are known by experts in the art.

Furthermore, the composition could comprise at least one pharmaceutically acceptable carrier.

The manufacturing of the pharmaceutical composition is performed according to standard procedures. Due to the stability of precepitated RNA it is possible to store RNA in dried state. Prior to the transfer of the RNA according to the invention the RNA e. g. is solved into an appropriate solution additionally comprising at least the basic peptide or protein and preferably further comprising stabilizing components, e.g. at least one RNase inhibitor.

According to the invention the complex is used for the manufacturing of a pharmaceutical composition for the stimulation of an immune response. As outlined above, the composition is used to introduce the genetic information for an antigen into the cell evoking an immune response directed against the certain antigen. For example, in the treatment of cancer this could be a very efficient strategy to overcome the disease. Also in the field of infections like AIDS, Hepatitis or Malaria, the use of the pharmaceutical composition, promises good results.

### Examples

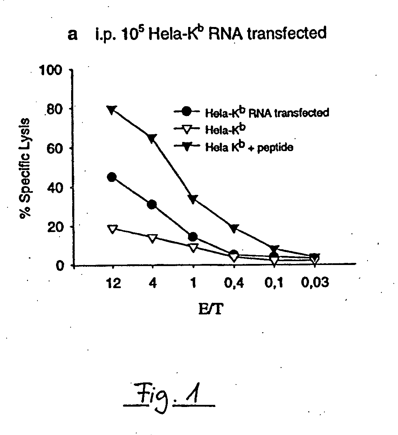

In figures 1 to 4, to which reference is made in the following description, the features as here listed are shown.
- Figure 1: Induction of β-Gal specific CTL (cytotoxic T lymphocytes) activity. B6 mice were immunized i.p. (intraperitoneal) with 10⁵ Hela K^{b} cells electroporated with βglacZβgαₙ RNA. Spleen cells were stimulated in vitro with the synthetic peptide ICPMYARV corresponding to the amino acid sequence 497-504 of E. coli β-galactosidase. CTL activity was determined in a standard ⁵¹Cr-release assay 5 d after in vitro restimulation. The targets were Hela K^{b} cells, Hela K^{b} cells transfected with βglacZβgαₙ RNA and Hela K^{b} cells incubated with synthetic peptide ICPMYARV.

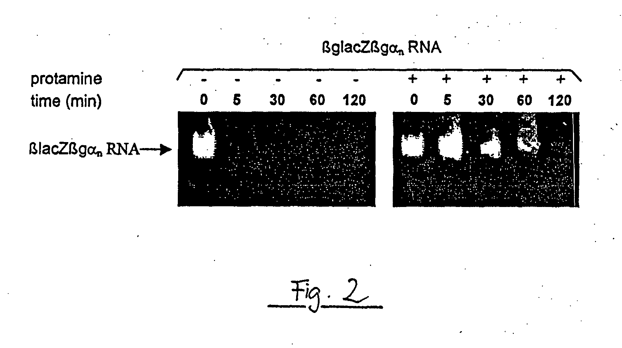

- Figure 2: RNA degradation protection assay. Arrowhead indicates 0.5 µg of naked βglacZβgαₙ RNA or protamine-condensed βglacZβgαₙ RNA,respectively. RNA was incubated with 2.5 % fetal bovine serum in vaccination buffer at room temperature. At the indicated time, samples were withdrawn, treated with RNase inhibitor, frozen at -70 °C and analyzed on a 1 % agarose gel.

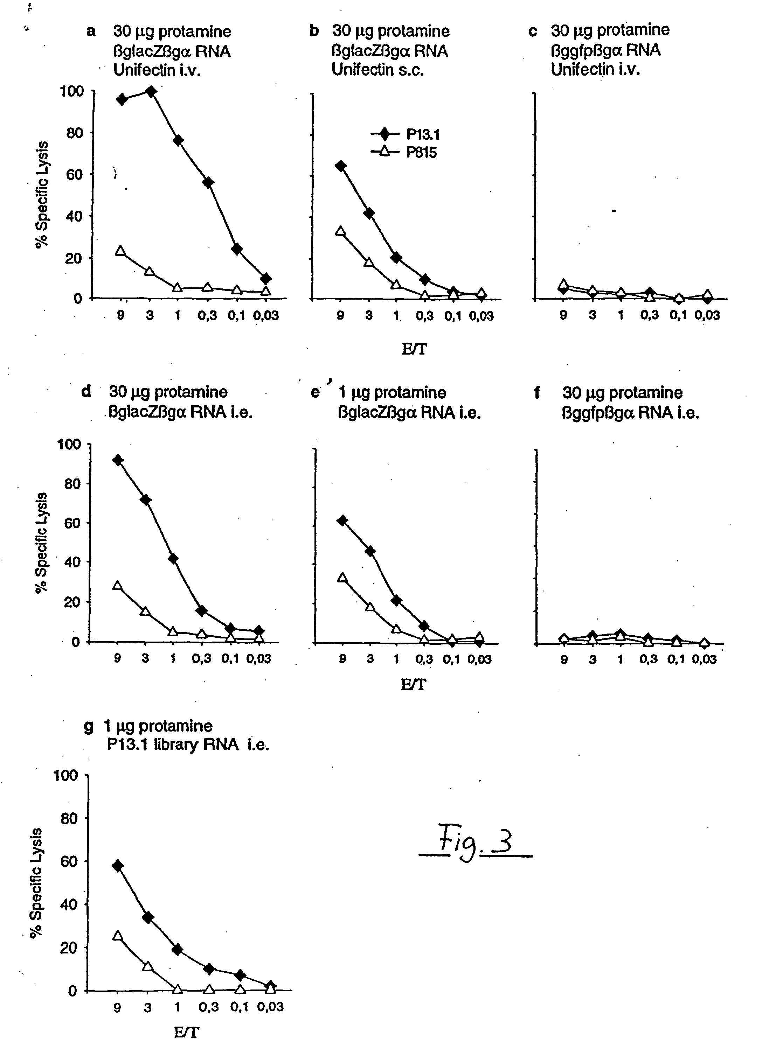

- Figure 3: Induction of β-Gal specific CTL activity. BALB/c mice were immunized as described with Unifectin-encapsulated protamine-condensed RNA or with protamine condensed RNA. 30 µg of protamine-βglacZβgαₙ RNA + Unifectin was injected intravenously (a) or subcutaneously (b) and 30 µg of protamine-βgfpβgαₙ RNA + Unifectin was injected intravenously as a control (c). Using the ear pinna (i.e.), 30 µg of protamine-βglacZβgαₙ RNA (d), 1 µg of βglacZβgαₙ protamine-RNA (e),30 µg of protamine-βgfpβgαₙ RNA as a control (f) and 30 µg of P13.1 protamine-library-RNA (g), respectively, were injected once. Spleen cells were stimulated twice in vitro with the synthetic peptide TPHPARIGL corresponding to the H2^{d} epitope E. coli β-Gal (amino acid 876-884 sequence). CTL activity was determined in a standard ⁵¹Cr-release assay 5 days after in vitro restimulation. The targets were P13.1 cells (H-2^{d} lacZ transfected) and P815 cells-(H-2^{d}).

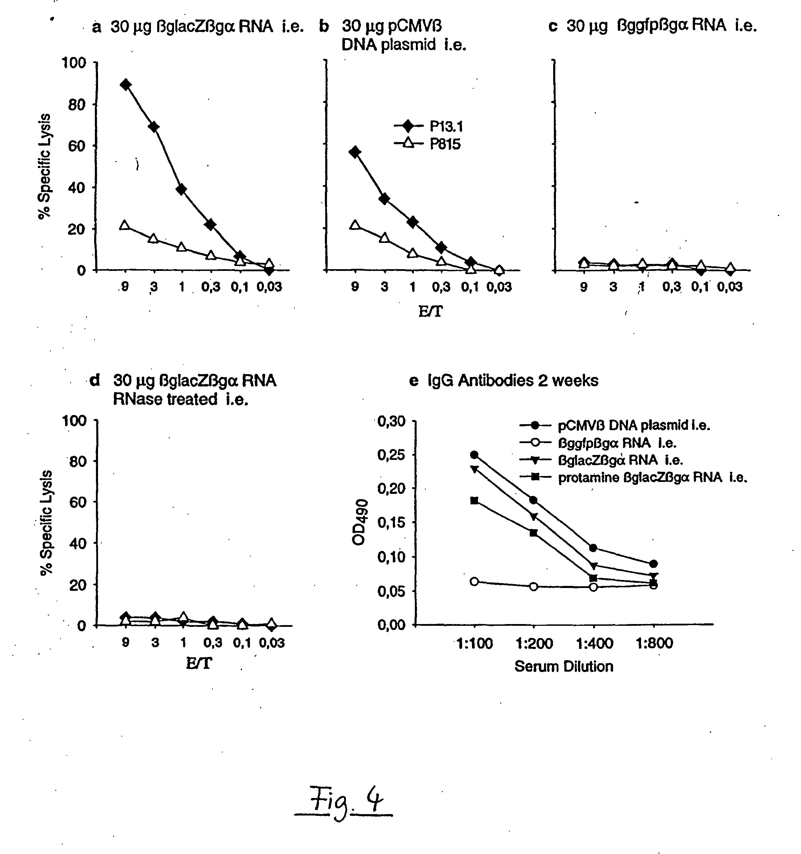

- Figure 4: Induction of β-Gal specific CTL activity. BALB/c mice were immunized i.e. with 30 µg of unprotected βglacZβαₙ RNA (a) or with 30 µg of pCMVβ DNA plasmid (b) as described in Ref. 10. Control mice were immunized i.e. with 30 µg of unprotected βgfpβgαₙ RNA (c) or with 30 µg of RNase treated βglacZβgαₙ RNA (d). Spleen cells were stimulated twice in vitro with the synthetic peptide TPHPARIGL. CTL activity was determined in a standard ⁵¹Cr-release assay 5 days after in vitro restimulation. The targets were P13.1 cells (H-2^{d} lacZ transfected) and P815 cells (H-2^{d}). The humoral IgG anti-β-Gal immune response was detected by ELISA from sera two weeks after immunization (e). Average values were estimated over the OD₄₉₀ Of 1:100 to 1:800 diluted sera. Mice were immunized once i.e. with 30 µg of pCMVβ DNA plasmid, 30 µg of βggfpβgαₙ RNA, 30 µg of unprotected βglacZβgαₙ RNA or 30 µg of protamine-βglacZβgαₙ RNA.

### Methods and results

### Plasmids and RNA synthesis

Capped βglacZβgαₙ RNA transcripts for vaccination containing Xenopus laevis β-globin sequences were prepared by in vitro transcription with Sp6 Cap-Scribe (Boehringer Mannheim) according to the manufacturer's directions from the linearized SpjC-βglacZβgαₙ, plasmid (a HindIII-BamHI fragment containing the lacZ gene was excised from pCH110 plasmid (Pharmacia) and inserted into the HindIII and BglII sites of the pSpjC-l plasmid, which was kindly provided by J. Lord, Warwick, UK). For a negative control βggfpβgαₙ RNA from the linearized pT7TS-βggfpβgαₙ plasmid was prepared containing the gfp gene from Aequoria victoria with T7 Cap-Scribe (Boehringer Mannheim; the SmaI-SpeI fragment of the gfp gene was excised from pCFP (Clontech) and inserted into the EcoRV and SpeI sites of pT7TS plasmid, which was generously provided by P. A. Krieg, Austin, Texas, USA). P13.1 library RNA transcripts were prepared from a linearized cDNA library from P13.1 cells with T3 Cap-Scribe (Boehringer Mannheim). The cDNA library was constructed from P13.1 cells using the ZAP-cDNA Synthesis Kit (Stratagene) according to the manufacturer's instructions. Prior to in vitro transcription, all linearized plasmids were extracted twice with phenolchloroform and precipitated with ethanol/sodium acetate.

RNA transcripts were treated with DNase (2 U/µg of DNA-template, Boehringer Mannheim) at 37 °C for 15 min and extracted with phenol/chloroform. Finally RNA was precipitated with ethanol/sodium acetate, solubilized in DEPC-treated water and stored at -80 °C until use.

### Immunizations with nucleic acids

Protamine (free base, grade IV from salmon) and the protamine derivates (protamine phosphate and protamine sulphate) were purchased from Sigma. The cationic liposome Unifectin (structure see below) was obtained from A. Surovoy, Tübingen.

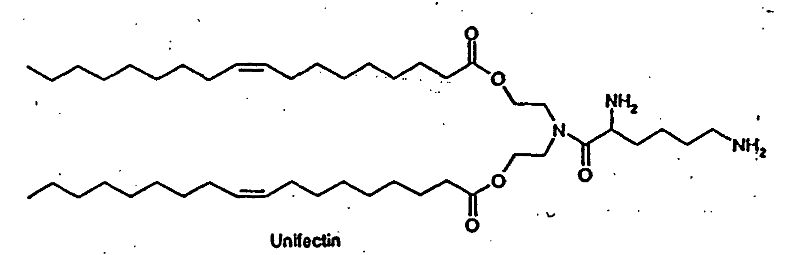

A protamine : RNA ratio of 1:2 throughout was incubated in 100 µl vaccination buffer (150 mM NaCl, 10 mM HEPES, pH 7.4) at room temperature for 5 min. Unifectin-encapsulated RNA was prepared in a liposome : protamine : RNA ratio of 2:1:1, whereas Unifectin was added 10 min after incubation of protamine-RNA in 60 µl vaccination buffer at room temperature. 100 µl vaccination solution was injected i.p. (intraperitoneal), i.v. (intravenous), s.c. (subcutaneous) and i.m. (intramuscular). Naked RNA and DNA plasmid controls were prepared in 100 µl DEPC treated vaccination buffer and applied immediately. 50 µl vaccination solution was injected into each ear pinna of anaesthetized mice (Metofane, Janssen).

### ELISA

Blood samples were obtained from the tail vein of mice. Sera were prepared and ELISA plates (Greiner) were coated overnight with β-galactosidase (Sigma) in PBS buffer at 4 °C.

After washing with 0.05 % Tween-20/PBS the plates were blocked with 1 % bovine serum albumin/PBS for 1 h at 37 °C. Sera were diluted in 0.05% Tween-20/PBS and administered to the plates for 1 h at 37°C. After further washing steps, bound antibodies were spotted with a peroxidase-conjugated goat anti-mouse IgG immunoglobulin (Sigma). The plates were washed and developed with 5-aminosalicylic acid (Sigma) in phosphate buffer and read at 490 nm.

### Cell culture and ⁵¹Cr release assay

P 815 and P13.1 cells (Ref. 18) were cultured in α-MEM containing 10 % FCS, 2-ME, L-glutamine, and antibiotics. 7-9 d after immunization of mice, recipient spleens were removed and the splenocytes were restimulated with synthetic β-Gal peptide TPHPARIGL (ICPMYARV for the Hela-K^{b} approach) at 50-100 mM. CTL (cytotoxic T lymphocyte) lines were generated by weekly restimulation with syngeneic, irradiated spleen cells and synthetic peptides as described in Ref. 19. Lysis of target cells was tested in a standard 5 h ⁵¹Cr-release assay described previously in Ref. 20. Where applicable, target cells were pulsed with peptide during the ⁵¹Cr labelling period. After incubation of effector and target cells in round-bottom 96-well plates for 5 h, 50 µl of 200 µl culture supernatant were removed and radioactivity was measured in a microplate format scintillation counter (1450 Microbeta Plus), using solid phase scintillation (Luma Plate-96, Packard). Percent specific release was determined from the amount of ⁵¹Cr released into the medium (A), corrected for spontaneous release (B), and compared to the total ⁵¹Cr content of the 1% Triton-X-lOO lysed target cells (C): % Specific lysis = 100(A-B) / (C-B) .

### RNA degradation protection assay

0.5 µg of βglacZβgαₙ RNA was incubated for 5 min in 5 µl vaccination buffer containing 0.25 µg of protamine. At time point 0.5 µl vaccination buffer containing 5 % fetal bovine serum was added and incubated at room temperature. At an indicated period of time, samples were withdrawn and mixed with 2 µl vaccination buffer containing 10 U of RNase inhibitor (Sigma) and frozen at -70 °C. 4 µl DEPC-treated vaccination buffer containing 10 U of proteinase K and 4 µl DEPC-treated vaccination buffer containing 20% SDS and loading dye was added. Samples were analyzed on a 1% agarose gel in DEPC-treated Tris-acetate-EDTA buffer.

To study the efficiency of RNA-based vaccines a plasmid vector for in vitro transcription of lacZ-RNA (βglacZβgαₙ RNA) was engineered containing a cap structure, a poly(A) tail and flanked at the 5' end and the 3' end by untranslated regions (UTRs) from B-globin of Xenopus laevis to enhance cytosolic protein expression (Refs 4 and 5).

To access its immunogenic potential, Hela-K^{b} cells (Ref 6) were electroporated with βglacZβg ₙ RNA (as follows: settings for Gene Pulser (Bio-Rad): Voltage: 850 V, Capacitance: 50 µF, Current: 50 mA, Resistance:∞ Ω. Cells (0.5 ml) were placed on ice for 5 minutes and transferred to 9.5 ml of complete α-MEM medium and incubated at 37°C over night) and injected i.p. into B6 (H2^{b}) mice. A specific CTL response against β-Gal after stimulation of spleen cells in vitro with the synthetic peptide ICPMYARV corresponding to the K^{b} presented epitope of E. coli β-galactosidase (amino acid sequence 497-504) (Ref. 7) was observed that was comparable to CTL induction upon immunization with in vitro RNA transfected dendritic cells (Ref. 8) (Fig. 1).

For direct injection of RNA into mice, protamine was used to condense and protect RNA from RNase degradation. Protamine is a small naturally occurring, arginine rich protein (Ref. 9) . As shown in Figure 2, the protamine RNA complex is less sensitive to RNase activity in 2.5 % fetal bovine serum (undegraded full length RNA was visible after 60 min incubation) as compared to naked RNA, which was degraded within minutes.

To generate a specific CTL response in vivo, 30 µg of prota-protamine-condensed βglacZβgαₙ RNA was encapsulated with a cationic liposome named Unifectin by a very simple procedure and injected intravenously and subcutaneously into BALB/c mice. A specific CTL response against β-Gal (Fig 3a, b) was obtained after stimulation in vitro with P13.1 cells (H2^{d}, lacZ transfected). Control mice were immunized intravenously with 30 µg of Unifectin-encapsulated protamine condensed βggfpβgαₙ RNA, encoding the gfp gene from Aequoria victoria (Fig. 3c). Intramuscular or intraperitoneal injection of liposome encapsulated RNA failed to raise specific CTL (data not shown). These results emphasize the importance of choosing the most suitable injection site for successful nucleic acid vaccination.

The efficiency of RNA vaccination using the ear pinna (i.e.) as injection site was investigated. Injection into the ear pinna of Unifectin-encapsulated protamine-condensed βglacZβgαₙ RNA did not generate specific CTL (data not shown), whereas a single injection of 30 µg protamine-condensed βglacZβgαₙ RNA without liposome triggered a specific CTL response that was recalled after stimulation in vitro using a synthetic peptide corresponding to the H2^{d} β-Gal epitope TPHPARIGL (amino acid sequence 876-884) (Ref. 11) (Fig. 3d). only 1 µg of protamine-condensed βglacZβgαₙ RNA (a low amount of nucleic acid compared to the regular protocol for DNA vaccination in mice that involve at least 50 µg of DNA (Refs 12 and 13)) sufficed for in vivo CTL priming (Fig. 3e). Control mice immunized i.e. with 30 µg of protamine condensed βggfpβgαₙ RNA did not develop anti-β-Gal CTL (Fig. 3f). After demonstration that it is possible to raise CTL in vivo directed against a single protein encoded by a homogenous RNA population, the assumption was explored that a complex mRNA mixture could trigger antigen-specific CTL as well. It has previously been shown that RNA transcribed in vitro from a cDNA library can be taken up by dendritic cells (DC) in vitro and that RNA-loaded DC induce CTL responses in vivo (Ref. 8). The possibility of triggering a CTL response directed against β-Gal by employing a complex mixture of RNA was evaluated using RNA transcribed in vitro from a cDNA library of P13.1 cells (H2^{d}, lacZ transfected). Immunization (i.e.) with protamine-condensed, P13.1 library-RNA, for example led to a specific CTL response against β-Gal (Fig. 3g). This is remarkable, since only a low amount of of lacZ encoding RNA can be expected in the 30 µg of total unfractionated P13.1 RNA.

To test whether protection of RNA by protamine is essential for in vivo CTL priming BALB/c mice were immunized i.e. with 30 µg of unprotected βglacZβgαₙ RNA immediately after preparation (Fig. 4a). Unexpectedly, naked RNA immunization also generated a β-Gal specific CTL response, that was even stronger than the injection i.e. of 30 µg of naked pCMVβ plasmid DNA, for example (Fig. 4b). On the contrary, injection of unprotected βggfpβgαₙ RNA, as well as RNase-treated βglacZβgαₙ RNA did not induce β-Gal specific CTL (Fig. 4c, Fig. 4d). Furthermore the injection of unprotected βglacZβgαₙ RNA stored for 1 h at room temperature in buffer that was not especially made RNase-free did not induce CTL (data not shown). The development of humoral immunity was tested by β-Gal ELISA plate assay using sera 2 weeks after immunization. IgG antibodies specific for β-Gal are detectable in sera of mice immunized i.e. either with 30 µg of pCMVβ plasmid DNA, 30 µg of unprotected βglacZβgαₙ RNA, or 30 µg of protamine-condensed βglacZβgαₙ RNA. Sera of mice injected i.e. with βggfpβgαₙ RNA was used as control (Fig. 4e).

These results allow conclusions for vaccine development that are wholly unexpected, especially with respect to therapeutic vaccination against cancer. Although the surprising efficiency of freshly prepared naked RNA for vaccination is unlikely to promote its use in a clinical setting, protamine-protected RNA appears to have all the advantages of DNA vaccination, but without the intrinsic risk of DNA integration into the genome and the induction of pathogenic anti-DNA antibodies (Ref. 14). Tumor antigens that are already known could be used as multi-epitopic RNA vaccines without regard for HLA expression in the vaccinee (as is required for peptide vaccines (Ref. 15)). Moreover, since heterogenous RNA populations lead to an antigen-specific CTL response, RNA can be prepared from a cDNA library established from a small tumor sample (Ref. 8). This approach seems to present a way to induce immunity against certain types of cancer in patients in which tumor rejection antigens have not been identified, although it bears the potential risk of inducing autoimmunity, as does any other complex, tumor-derived vaccine, such as gp96 (Ref. 16).

Immunization into the ear pinna was highly efficient. Ear pinna tissue was tested 24 h after injection with protamine-condensed βglacZβgαₙ RNA for expression of the lacZ gene by X-gal staining. The intensity of β-gal expression was strong and not distinguishable from that of a control pCMV-β DNA plasmid (data not shown). In addition, the reason for the expression of RNA which occurs so readily in an immunogenic way, needs to be elucidated (RT-PCR confirmed that βglacZβgαₙ RNA was present in the ear pinna for more than 48 h after injection (data not shown)). One possibility is that RNA transduces dendritic cells directly in vivo. It is certainly an advantage that the in vivo uptake of RNA is mediated without the need for any adjuvants or transfection vehicles such as cationic liposomes, which often cause latent toxicity in higher doses. Cationic liposomes are highly toxic toward macrophages and induce a drop in the production of at least two immunomodulators by activated macrophages (Ref. 17). The RNA-protective protamine does not appear to be immunogenic since no protamine-specific IgG antibodies were detected in sera of mice (data not shown). For practical purpose, RNA can be stored in precipitated form and solubilized in transfection buffer containing protamine directly before vaccination.

The results of the invention can be summarized as follows. To study the efficiency of RNA based vaccines, RNA coding for the model antigen β-galactosidase (β-Gal) was transcribed in vitro from a lacZ gene flanked by stabilizing Xenopus laevis β-globin 5' and 3' sequences and was protected from RNase degradation by condensation with the polycationic peptide protamine. The liposome-encapsulated condensed RNA-peptide complex, the condensed RNA-peptide complex without liposome or naked, unprotected RNA, was injected into BALB/c (H2^{d}) mice. All preparations led to protein expression in the local tissue, activation of H-2-restricted anti-β-Gal cytotoxic T lymphocytes (CTL) and production of IgG antibodies reactive against β-Gal. RNA-triggered CTL were even more efficient in the lysis of lacZ transfected target cells as CTL triggered by a lacZ DNA eukaryotic expression vector. Immunization with RNA transcribed from a cDNA library from the β-gal expressing cell line P13.1 again led to β-Gal specific CTL and IgG induction. Thus, both naked and especially protected RNA can be used to elicit a specific immune response, whereby the protected RNA is stable in vitro for a longer period of time. RNA vaccines can be produced in high amounts and have the same major advantages as DNA vaccines but lack the potentially harmful effect of DNA integration into the genome.

### References

1) Liu, M. A., Fu, T. M., Donnelly, J. J., Caulfield, M. J.& Ulmer, J. B. DNA vaccines. Mechanisms for generation of immune responses. Adv. Exp. Med. Biol. 452, 187-191 (1998).
2) Wolff, J. A., Malone, R. W., Williams, P., Chong, W., Acsadi, G. & Felgner, P. L. Direct gene transfer into mouse-muscle in vivo. Science 257, 1465-1468 (1990).
3) Martinon, F., Krishnan, S., Lenzen, G., Magné, R., Gomard, E., Guillet, J-G., Lévy J-P. & Meulien, P. Induction of virus specific cytotoxic T lymphocytes in vivo by liposome entrapped mRNA. Eur. J. Immunol. 23, 1719-1722 (1993).
4) Malone, R. W., Felgner, P. L. & Verma, I. Cationic liposome-mediated RNA transfection. Proc. Natl. Acad. Sci. USA 86, 6077-6081 (1989).
5) Strong, T. V., Hampton, T. A., Louro, I., Bilbao, G., Conry, R. M. & Curiel,. D. T. Incorporation of β-globin untranslated regions into a sihdbis virus vector for augmentation of heterologous mRNA expression. Gene Ther. 4, 624-627 (1997).
6) Falk, K., Rötzschke, 0., Faath, S., Goth, S., Graef, I., Shastri, N. & Rammensee, H.-G. Both human and mouse cells expressing H-2Kb and ovalbumin process the same peptide, SIINFEKL. Cell Immunol. 150, 447-52 (1993).
7) Oukka, M., Cohen-Tannoudji, M., Tanaka, Y., Babinet, C & Kosmatopoulos, K. Medullary thymic epithelial cells induce tolerance to intracellular proteins. J. Immunol. 156, 968-975 (1996).
8) Boczkowski, D., Nair, S. K., Snyder, D. & Gilboa, E. Dendritic cells pulsed with RNA are potent antigen-presenting cells in vitro and in vivo. J. Exp. Med. 184, 465-472 (1996).
9) Warrant, R. W. & Kim, S. H. Alpha-Helix-double helix interaction shown in the structure of a protamine-transfer RNA complex and a nucleoprotamine model. Nature 27, 130-135 (1978).
10) Förg, P., von Hoegen, P., Dalemans, W. & Schirrmacher V. Superiority of the ear pinna over muscle tissue as site for DNA vaccination. Gene Ther. 5, 789-797 (1998).
11) Gavin, M.A., Gilbert, M. J., Riddell, S. R., Greenberg, P. D. & Bevan M. J. Alkali hydrolysis of recombinant proteins allows for the rapid identification of class I MHC-restricted CTL epitopes. J. Immunol. 151, 3971-3980 (1993).
12) Montgomery, D. L. et al. Heterologous and homologous protection against influenza A by DNA vaccination: Optimization of DNA vectors. DNA and Cell Biology 12, 777-783 (1993).
13) Martins, L. P., Lau, L. L., Asano, M. S. & Ahmed, R. DNA vaccination against persistent viral infection. J. Virol. 69, 2574-2582 (1995).
14) Gilkeson G. S., Pippen, A. M. & Pisetsky, D. S. Induction of cross-reactive anti-dsDNA antibodies in preautoimmune NZB/NZW mice by immunization with bacterial DNA. J. Clin. Invest. 95, 1398-1402 (1995).
15) Deres, K., Schild, H., Wiesmüller, K-H., Jung, G. & Rammensee, H.-G. In vivo priming of virus-specific cytotoxic T lymphocytes with synthetic lipopeptide vaccine. Nature 342, 561-564 (1989).
16) Tamura, Y., Peng, P., Liu, K., Daou, M. & Srivastava, P. K. Immunotherapy of tumors with autologous tumor-derived heat shock protein preparations. Science 278, 117-120 (1997).
17) Filion, M. C. & Phillips, N. C. Toxicity and immunomodulatory activity of liposomal vectors formulated with cationic lipids toward immune effector cells. Biochim. Biophys. Acta 1329, 245-356 (1997).
18) Carbone, F. R. & Bevan, M. J. Class I-restricted processing and presentation of exogenous cell-associated antigen in vivo. J.Exp. Med. 171, 377-387 (1990).
19) Schild, H., Norda, M., Deres, K., Falk, K., Rotzschke, O., Wiesmüller, K.-H., Jung, G. & Rammensee, H.-G. Fine specifity of cytotoxic T lymphocytes primed in vivo either with virus or synthetic lipopeptide vaccine or primed in vitro with peptide. J. Exp. Med. 174, 1665-1668 (1991).
20) Rammensee, H.-G., Schild, H. & Theopold, U. Protein-specific cytotoxic T lymphocytes. Recognition of transfectants expressing intracellular, membrane-associated or secreted forms of β-galactosidase. Immunogenetics 30, 296-302 (1989).
21) Conolly, J. H. Effect of Histones and Protamine on the Infectivity of Semliki Forest Virus and its Ribonucleic Acid. Nature 19, 858 (1966).
22) Dubes, G. R., and Wegrzyn, R. J. Rapid Ephemeral Cell Sensitization as the Mechanism of Histone-Induced and Protamine-Induced Enhancement of Transfection by Poliovirus RNA. Protoplasma 96, 209-223 (1978).
23) Ying, H., Zaks, T.Z., Wang, R., Irvine, K. R., Kammula, U. S., Marincola, F. M., Leitner, W. W. & Restifo, N. P. Cancer therapy using a self-replicating RNA vaccine. Nature medicine 5, 823-827 (1999).

## Claims

1. Use of a composition comprising at least one complex comprising at least one mRNA and at least one cationic, especially polycationic, peptide or protein for the preparation of a vaccine.

2. Use of a composition according to claim 1, whereby the composition does not comprise an adjuvant or transfection, vehicle.

3. Use of a composition according to claim 1or 2, **characterized in that** the peptide or protein is a nucleic acid binding peptide or proteins particularly protamine, poly-L-lysine and/or a histone.

4. Use of a composition according to one of claims 1 to 3, **characterized in that** the mRNA encodes at least one antigen.

5. Use of a composition according to one of claims 1 to 4, **characterized in that** it comprises at least one factor in order to further stabilize the mRNA.

6. Use of a composition according to one of claims 1 to 5, **characterized in that** the mRNA comprises sequences, especially at the 5'- and/or the 3'-terminal end, which are essentially not translatable during expression.

7. Use of a composition according to one of claims 1 to 6, **characterized in that** the mRNA comprises at least one internal ribosomal entry site.

8. Use of a composition according to one of claims 1 to 7, **characterized in that** the mRNA is a collection of mRNAs.

9. Use of a composition according to one of claims 1 to 8, **characterized in that** it is a pharmaceutical composition comprising preferably at least one pharmaceutically acceptable carrier.

10. Use of a composition according to one of claims 1 to 9 for the manufacturing of a vaccine for the treatment of infectious diseases, especially viral infections.

11. Use of a composition according to claim 10 for the preparation of a vaccine for the treatment of AIDS, Hepatitis or Malaria.

12. Use of a composition according to one of claims 1 to 9 for the preparation of a vaccine for the stimulation of an immune response.

13. Use of a composition according to one of claims 1 to 9 for the preparation of a vaccine for the treatment of cancer.

## Patentansprüche

1. Verwendung einer Zusammensetzung umfassend mindestens einen Komplex, umfassend mindestens eine mRNA und mindestens ein kationisches, insbesondere ein polykationisches Peptid oder Protein zur Herstellung eines Vakzins.

2. Verwendung einer Zusammensetzung gemäß Anspruch 1, wobei die Zusammensetzung kein Adjuvanz- oder Transfektionsvehikel umfasst.

3. Verwendung einer Zusammensetzung gemäß Anspruch 1 oder 2, **dadurch gekennzeichnet, dass** das Peptid oder Protein ein nukleinsäurebindendes Peptid oder Protein ist, insbesondere Protamin, Poly-L-Lysin und/oder ein Histon.

4. Verwendung einer Zusammensetzung gemäß einem der Ansprüche 1 bis 3, **dadurch gekennzeichnet, dass** die mRNA mindestens ein Antigen kodiert.

5. Verwendung einer Zusammensetzung gemäß einem der Ansprüche 1 bis 4, **dadurch gekennzeichnet, dass** sie mindestens einen zur weiteren Stabilisierung der mRNA umfasst.

6. Verwendung einer Zusammensetzung gemäß einem der Ansprüche 1 bis 5, **dadurch gekennzeichnet, dass** die mRNA, insbesondere am 5'- und/oder am 3'terminalen Ende, Sequenzen umfasst, die während der Expression im Wesentlichen nicht translatierbar sind.

7. Verwendung einer Zusammensetzung gemäß einem der Ansprüche 1 bis 6, **dadurch gekennzeichnet, dass** die mRNA mindestens eine interne ribosomale Angriffsstelle umfasst.

8. Verwendung einer Zusammensetzung gemäß einem der Ansprüche 1 bis 7, **dadurch gekennzeichnet, dass** die mRNA eine Sammlung an mRNAs ist.

9. Verwendung einer Zusammensetzung gemäß einem der Ansprüche 1 bis 8, **dadurch gekennzeichnet, dass** sie eine pharmazeutische Zusammensetzung ist, die bevorzugt mindestens einen pharmazeutisch geeigneten Träger umfasst.

10. Verwendung einer Zusammensetzung gemäß einem der Ansprüche 1 bis 9 zur Herstellung eines Vakzins zur Behandlung von infektiösen Erkrankungen, insbesondere von viralen Infektionen.

11. Verwendung einer Zusammensetzung gemäß Anspruch 10 zur Herstellung eines Vakzins zur Behandlung von AIDS, Hepatitis oder Malaria.

12. Verwendung einer Zusammensetzung gemäß einem der Ansprüche 1 bis 9 zur Herstellung eines Vakzins zur Stimulierung einer Immunantwort.

13. Verwendung einer Zusammensetzung gemäß einem der Ansprüche 1 bis 9 zur Herstellung eines Vakzins zur Behandlung von Krebs.

## Revendications

1. Utilisation d'une composition comprenant au moins un complexe comprenant au moins un ARNm et au moins un peptide ou une protéine cationique, spécialement polycationique pour la préparation d'un vaccin.

2. Utilisation d'une composition selon la revendication 1,
au moyen de laquelle la composition ne comprend pas d'adjuvant ni de véhicule de transfection.

3. Utilisation d'une composition selon la revendication 1 ou 2,
**caractérisée en ce que** le peptide ou la protéine est un peptide ou une protéine liant à un acide nucléique, particulièrement une protamine, une poly-L-lysine et/ou une histone.

4. Utilisation d'une composition selon l'une des revendications 1 à 3,
**caractérisée en ce que** l'ARNm code au moins un antigène.

5. Utilisation d'une composition selon l'une des revendications 1 à 4,
**caractérisée en ce qu'**elle comprend au moins un facteur de stabilisation supplémentaire de l'ARNm.

6. Utilisation d'une composition selon l'une des revendications 1 à 5,
**caractérisée en ce que** l'ARNm comprend des séquences, spécialement à l'extrémité 5'- et/ou 3' terminal, qui sont essentiellement non traduisibles durant l'expression.

7. Utilisation d'une composition selon l'une des revendications 1 à 6,
**caractérisée en ce que** l'ARNm comprend au moins un site d'entrée interne du ribosome.

8. Utilisation d'une composition selon l'une des revendications 1 à 7,
**caractérisée en ce que** l'ARNm est un ensemble d'ARNm.

9. Utilisation d'une composition selon l'une des revendications 1 à 8,
**caractérisée en ce qu'**elle est une composition pharmaceutique comprenant de préférence au moins un support pharmaceutiquement acceptable.

10. Utilisation d'une composition selon l'une des revendications 1 à 9 pour préparation d'un vaccin pour le traitement de maladies infectieuses, spécialement d'infections virales.

11. Utilisation d'une composition selon la revendication 10 pour la préparation d'un vaccin pour le traitement du SIDA, de l'hépatite ou du paludisme.

12. Utilisation d'une composition selon l'une des revendications 1 à 9 pour la préparation d'un vaccin pour la stimulation d'une réponse immune.

13. Utilisation d'une composition selon l'une des revendications 1 à 9 pour la préparation d'un vaccin pour le traitement du cancer.
